(19) **Europäisches Patentamt / European Patent Office / Office européen des brevets**

(11) **EP 1 769 809 A2**

(12) # EUROPEAN PATENT APPLICATION

(43) Date of publication:
04.04.2007 Bulletin 2007/14

(51) Int Cl.:
*A61L 12/08* (2006.01)  *G02B 1/04* (2006.01)
*G02C 7/04* (2006.01)

(21) Application number: 06076332.3

(22) Date of filing: 21.11.2003

(84) Designated Contracting States:
**DE FR GB IE IT**

(30) Priority: **22.11.2002 US 428620 P**
**18.11.2003 US 715903**

(62) Document number(s) of the earlier application(s) in accordance with Art. 76 EPC:
**03789934.1 / 1 599 236**

(71) Applicant: **Johnson & Johnson Vision Care, Inc.**
**Jacksonville, FL 32256 (US)**

(72) Inventors:
• **Rathore, Osman**
**Jacksonville, FL 32256 (US)**
• **Zanini, Diana**
**Jacksonville, FL 32277 (US)**

• **Neely, Frank**
**Jacksonville, FL 32277 (US)**
• **Riederer, Donald**
**Jacksonville,**
**Florida 32258 (US)**
• **Andersson, Ann Margaret**
**Hillsborough, NJ 08844 (US)**
• **Vanderlaan, Douglas**
**Jacksonville, FL 32206 (US)**

(74) Representative: **Mercer, Christopher Paul et al**
**Carpmaels & Ransford**
**43-45 Bloomsbury Square**
**London WC1A 2RA (GB)**

Remarks:
This application was filed on 30 - 06 - 2006 as a divisional application to the application mentioned under INID code 62.

(54) **Antimicrobial lenses displaying extended efficacy, processes to prepare them and methods of their use**

(57)     This invention relates to antimicrobial ophthalmic devices containing silver releasing compounds and methods for their production. The antimicrobial lenses of the present invention have percent haze of less than about 200% and have a release rate constant, calculated using a first order kinetics equation of up to about 1 days$^{-1}$ and an initial silver concentration of at least about 10 ppm.

EP 1 769 809 A2

**Description**

RELATED APPLICATIONS

This patent application claims priority of a provisional application, U.S. Ser. No. 60/428,620 which was filed on November 22, 2002.

FIELD OF THE INVENTION

This invention relates to contact lenses which provide controlled release of silver ions as well as methods of their production, and use.

BACKGROUND OF THE INVENTION

Contact lenses have been used commercially to improve vision since the 1950s. The first contact lenses were made of hard materials. They were used by a patient during waking hours and removed for cleaning. Current developments in the field gave rise to soft contact lenses, which may be worn continuously, for several days or more without removal for cleaning. Although many patients favor these lenses due to their increased comfort, these lenses can cause some adverse reactions to the user. The extended use of the lenses can encourage the buildup of bacteria or other microbes, particularly, *Pseudomonas aeruginosa,* on the surfaces of soft contact lenses. The build-up of bacteria and other microbes can cause adverse side effects such as contact lens acute red eye and the like. Although the problem of bacteria and other microbes is most often associated with the extended use of soft contact lenses, the build-up of bacteria and other microbes occurs for users of hard contact lens wearers as well.

US 5,820,918 discloses medical devices made from a water absorbable polymer material with a medical compound having low solubility in aqueous solutions such as an antiseptic or radiopaque compound. However, the procedures disclosed in the examples yield opaque devices which are not suitable for ophthalmic devices such as contact lenses.

Therefore, there is a need to produce contact lenses that inhibit the growth of bacteria or other microbes and/or the adhesion of bacterial or other microbes on the surface of contact lenses. Further there is a need to produce contact lenses which do not promote the adhesion and/or growth of bacteria or other microbes on the surface of the contact lenses. Also there is a need to produce contact lenses that inhibit adverse responses related to the growth of bacteria or other microbes. This need is filled by the invention described below.

BRIEF DESCRIPTION OF THE DRAWINGS

Figure 1 is a graph showing the amount of silver released from a contact lens containing AgI as a function of time.
Figure 2 is a graph showing the amount of silver released from a contact lens containing AgCl as a function of time.
Figure 3 is a graph showing the amount of silver released from a contact lens containing AgI as a function of time.

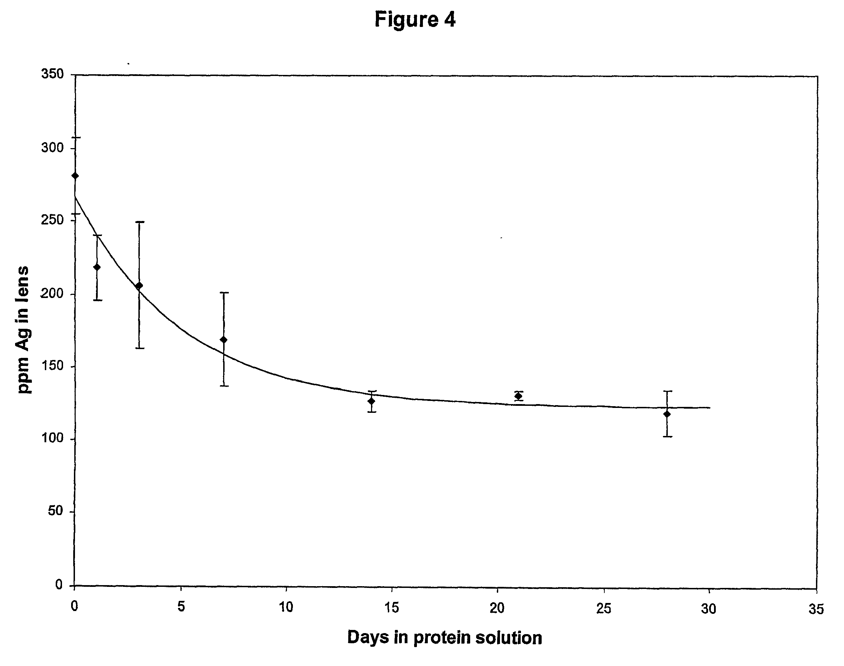

Figure 4 is a graph showing the amount of silver released from a contact lens containing Ag-imidazole as a function of time.
Figure 5 is a graph showing the amount of silver released from a contact lens containing silver 2-methylbenzenethiol, silver 2-aminothiophenol, and silver thiosalicylic acid as a function of time

DETAILED DESCRIPTION OF THE INVENTION

This invention includes an antimicrobial lens which is substantially free from haze comprising, consisting essentially of, or consisting of a contact lens comprising a polymer and at least one silver releasing compound, wherein silver is released from said contact lens during use, has a rate constant, calculated using a first order kinetics equation, of up to about 1 days$^{-1}$ and an initial silver concentration of at least about 10 ppm.

As used herein, the term, "antimicrobial lens" means a lens that exhibits one or more of the following properties, the inhibition of the adhesion of bacteria or other microbes to the lenses, the inhibition of the growth of bacteria or other microbes on lenses, and the killing of bacteria or other microbes on the surface of lenses or in an area surrounding the lenses. For purposes of this invention, adhesion of bacteria or other microbes to lenses, the growth of bacteria or other microbes on lenses and the presence of bacterial or other microbes on the surface of lenses are collectively referred to as "microbial colonization." Preferably, the lenses of the invention exhibit at least a about >0.25 log reduction, more preferably about >0.5 log reduction, most preferably greater than about 1.0 log reduction ($\geq$ 90% inhibition) of viable bacteria or other microbes. Such bacteria or other microbes include but are not limited to those organisms found in the eye, particularly *Pseudomonas aeruginosa, Acanthamoeba species, Staphyloccus. aureus, E. coli, Staphyloccus epidermidis,* and *Serratia marcesens.*

The present invention relates to contact lenses that display extended release of silver ions. As used herein extended release means release of silver ions in an amount sufficient to inhibit microbial colonization over an extended period of time, such as two days, preferably seven days, more preferably 14 days and in some cases as many as or more than 30 days. Thus, the present invention allows for the manufacture of ophthalmic devices that provide resistance to microbial colonization over their entire wear schedule for the ophthalmic device. It has been found that by careful selection of the amount and type of silver releasing compound that is incorporated in to the ophthalmic device, the duration over which silver ions are released may be modulated.

Chemical reaction kinetic models known to those skilled in the art may be used to describe the first order release of silver ions from a lens. Some of these models are described for example in Physical Chemistry, Fourth Edition, by F. Daniels and R. Alberty, John Wiley & Sons, Inc., New York, pp. 300-346. For example, the rate of silver release may be fitted to first order kinetics. In a first order release model, the rate of silver release varies proportionately to the total amount of silver remaining at any given time. This can be stated with the equation:

$$-d[Ag]/dt = k[Ag]$$

where $-d[Ag]/dt$ is the rate of change of the silver content in the lens, expressed in units of concentration/time, $[Ag]$ is the concentration of silver at any given time, and k is the first order rate constant. This equation can be rewritten as:

$$[Ag] = [Ag]_o e^{-kt}$$

where $[Ag]_o$ is the initial concentration of silver in the lens when the lens is placed in the eye, in contact with tear fluid, or in contact with an artificial tear fluid model, and t is the residence time of the lens on the eye, in contact with tear fluid, or in contact with an artificial tear fluid model.

The rate constant, k, is determined using experimental methods. The release of silver from the lens may be measured by placing a series of lenses having the same composition and formed by the same process in an artificial tear solution. Lenses are placed into fresh artificial tear solution each day or removed from the tear solution each day and analyzed via Instrumental Neutron Activation Analysis (INAA) or a like method to determine their silver content. Using data fitting software such as SigmaPlot 8.0, the average silver at each time interval is plotted versus the time of exposure. An exponential trendline is fitted to the data. Preferably the $R^2$ value for the trendline is relatively high, such as greater than about 0.80, and preferably greater than about 0.90. The exponential fit provides the rate constant k value, which has units of time $^{-1}$. Another gauge of the precision with which the k value is calculated is the standard error of the k value, which is also calculated with data fitting software.

In expressing the release behavior of silver from a contact lens using the above described kinetics model, it has been found that some lenses have an apparent constant amount of silver which does not release, releases very slowly even after 30 days, or may release only under different conditions. This silver is referred to herein as "nonreleasing silver". Because this silver is not released during the intended wear cycle for the lens, the non-releasing silver is subtracted from the lens silver constant at each time interval. In such cases, the first order rate model may be used to fit the release of the rest of the silver, referred to herein as "releasable silver". Thus the data is fit to the equation:

$$[Ag] = [Ag]_{NR} + [Ag]_o e^{-kt}$$

where $[Ag]$ is the total amount of silver in the lens at any time t, $[Ag]_{NR}$ is the concentration of nonreleasing silver in the lens, and $[Ag]_o$ is the initial amount of releasable silver.

Ophthalmic devices of the present invention display k values of up to about 1 days$^{-1}$, preferably between about 0.001 to about 0.5 days$^{-1}$ and more preferably between about 0.001 to about 0.3 days$^{-1}$. Ophthalmic devices that are intended for longer wear schedules (such as two or four week disposable or continuous wear lenses) will preferably have lower k values than ophthalmic devices which are intended for daily disposable wear. Also, therapeutic lenses, which are meant to kill an existing infection, may have an initial k value which is higher than the ranges specified above, but a "maintenance" k value within the ranges specified above. This may be achieved through the use of a single silver releasing compound or a mixture of silver releasing compounds.

Initial concentrations of silver will depend upon the amount of silver ion which is desired to be released and the haze of the resulting ophthalmic device. Suitable concentrations include at least about 10 ppm, preferably between about 10 and 10,000 ppm, more preferably between about 25 and 5,000 ppm and most preferably between about 50 and 3,000 ppm Ag, based upon the dry weight of ophthalmic device.

It has been found that the release of silver ions from an ophthalmic device may be modulated by controlling (a) the solubility of the silver releasing compound, the (b) electron density of the atom bound to the silver ion and (c) the initial concentration of the silver incorporated into the ophthalmic device.

With respect to solubility, silver releasing compounds which are suitable for use in the ophthalmic devices of the present invention include those having molar solubility of the silver ion in pure water at about 25°C is greater than about $2.0 \times 10^{-30}$ moles/L to about less than about 2 moles/L. With respect to the solubility, the preferred silver releasing compounds are silver releasing compounds where the silver ion has a molar solubility of greater than about $2.0 \times 10^{-17}$ moles/L.

As used herein, the term "pure" refers to the quality of the water used as defined in the CRC Handbook of Chemistry and Physics, 74th Edition, CRC Press, Boca Raton Florida, 1993. The term "molar solubility" refers to the number of moles of metal dissolved or dissociated from the anion per liter of water. This number is derived from the solubility-product constant ($K_{sp}$) measured in pure water at 25°C. (See Skoog, D.A. et al. FUNDAMENTALS OF ANALYTICAL CHEMISTRY, Fifth Edition, Saunders College Publishing, New York, 1988, *see also,* published values in CRC Handbook of Chemistry and Physics, 74th Edition, CRC Press, Boca Raton Florida, 1993) For example, if the silver releasing compound is silver carbonate ($Ag_2CO_3$), the $K_{sp}$ is expressed by the following equation

$$Ag_2CO_3(s) \rightarrow 2Ag^+(aq) + CO_3^{2-}(aq)$$

The $K_{sp}$ is calculated as follows

$$K_{sp} = [Ag^+]^2\,[CO_3^{-2}]$$

As silver carbonate dissolves, there is one carbonate anion in solution for every two silver cations, $[CO_3^{2-}] = \frac{1}{2}[Ag^+]$, and the solubility-product constant equation can be rearranged to solve for the dissolved silver concentration as follows

$$K_{sp} = [Ag^+]^2(1/2[Ag^+]) = \frac{1}{2}[Ag^+]^3$$

$$[Ag^+] = (2K_{sp})^{1/3}$$

The $K_{sp}$ may be used to calculate the molar solubility of any silver releasing compound as follows

$$\text{For AgX:}\quad [M] = (K_{sp})^{1/2}$$

$$\text{For Ag}_2\text{X:}\quad [M] = (2K_{sp})^{1/3}$$

$$\text{For Ag}_3\text{X:}\quad [M] = (3K_{sp})^{1/4}$$

It has been discovered that silver releasing compounds wherein the metal ion has a molar solubility of about greater than about $2.00 \times 10^{-30}$ moles/L to less than about 2 moles/L when measured at 25°C will continuously release the silver from lenses for a period of time from at least one day to up to or longer than a thirty day period. A preferred class of silver releasing compounds are silver salts, wherein the molar solubility of the silver ion is greater than or equal to about $2.00 \times 10^{-17}$ moles/L. The preferred molar solubility is greater than or equal to about $9.00 \times 10^{-9}$ moles/L to less than or equal to 1.0 x $10^{-5}$ moles/L when measured at 25°C.

As use herein, the term "silver releasing compound" means any compound which has the ability to bind silver and release it according to the rates specified herein. Suitable silver releasing compounds include silver salts wherein suitable anions include but are not limited to $PO_4^{-3}$, $Cl^{-1}$, $Br^{-1}$, $I^{-1}$, $S^{-2}$, $O^{-2}$ and the like. As used herein the term silver releasing compound does not include zeolites, such as those disclosed in WO03/11351. Examples of silver salts include but are not limited to silver sulfate, silver iodate, silver phosphate, silver sulfide, silver chloride, silver iodide, and silver oxide. The preferred silver salts are silver iodide and silver chloride.

In order to produce lenses having a clarity suitable for ophthalmic purposes, it is preferred that when the silver releasing compound is added to the lens formulation as a particulate, the diameter of the silver releasing compound be less than about ten microns (10 $\mu$m), more preferably less than about 5 $\mu$m, most preferably equal to or less than about 200 nm. The particle size may also effect the k value, so when the silver releasing compound is added to the lens formulation as a particulate, or precipitated into a lens, care should be taken to control the particle size.

Silver releasing compounds may also include silver complexes so long as the k value is within the aforementioned ranges. The term "silver complex" means any molecule or compound which contains non-bonding electrons capable of forming coordinate bonds to silver or groups capable of forming covalent bonds to silver. Preferred are those with multiple pairs of non-bonding electrons or groups which are able to form chelate structures with the silver ion. Examples of silver complexes include porphyrin compounds such as meso-tetraphenylporphyrin, vinyl pyridine, EDTA, thiols, crown ethers, thio crown ethers mixtures thereof and the like.

When using a silver complex, k values may be decreased by increasing the electron density at or near the atom bound to silver. Conversely, k value may be increased by decreasing the electron density at or near the atom bound to silver. The electron density at or near the atom bound to silver may be increased by adding electron donating groups, such as alkyl groups, amino groups, alkyl oxides, alkoxy groups, alcohols and the like. Similarly, the electron density of the atom bound to silver may be decreased by adding electron withdrawing groups such as carboxylic acids, sulfonates, amides, ammonium, nitro, cyano, acyloxy, and halo groups and the like. It will be appreciated that the electron donating and withdrawing groups may also be placed at other locations in the silver complex to achieve the desired effect on electron density of the atom attached to silver.

The amount of silver in the lenses is also critical to the amount of metal which will be released. For silver releasing compounds with low k values, higher amounts of silver may be used. So for example, for silver releasing compounds having k values less than about 0.2 it is desirable to add the silver releasing compound in an amount sufficient to provide an initial silver concentration of at least about 100 ppm. It will be appreciated that for metal compounds with low solubility, higher amounts of metal in the lens may be tolerated, as the release will be slow. Conversely, when using a silver releasing compound with a high solubility lesser amounts of metal would be used. With respect to adding silver releasing compounds, the molecular weight of the silver releasing compound determines the conversion of weight percent of metal ion to silver releasing compound.

Throughout the specification reference has been made to silver releasing compounds. However, those of skill in the art, will appreciate that the teachings of the present invention can be applied to antimicrobial metals other than silver. Other suitable antimicrobial metals include $Mg^{+2}$, $Zn^{+2}$, $Cu^{+1}$, $Cu^{+2}$, $Au^{+2}$, $Au^{+3}$, $Au^{+1}$, $Pd^{+2}$, $Pd^{+4}$, $Pt^{+2}$, $Pt^{+4}$, mixtures thereof and the like. Suitable counterions or ligands, k values and solubilities may be readily determined using the teachings of the present invention.

Preferred ophthalmic devices include soft contact lenses. Soft contact lenses are made from silicone elastomers or hydrogels, which include but are not limited to silicone hydrogels, and fluorohydrogels. Preferably, the lenses of the invention are optically clear, with optical clarity comparable to lenses such as lenses made from etafilcon A, genfilcon A, lenefilcon A, polymacon, acquafilcon A, balafilcon A, galyfilcon A, senofilcon A and lotrafilcon A.

The antimicrobial lenses of the present invention are also substantially free from unwanted haze. Specifically, lenses of the present invention have a percent haze that is less than about 200%, preferably less than about 150% and more preferably less than about 100%. Percent haze is measured using the following method. The haze is measured by placing a hydrated test lens in borate buffered saline in a clear 20 x 40 x 10 mm glass cell at ambient temperature above a flat black background illuminating from below with a fiber optic lamp (Titan Tool Supply Co. fiber optic light with 0.5" diameter light guide set at a power setting of 4-5.4) at an angle 66° normal to the lens cell, and capturing an image of the lens from above from above, normal to the lens cell with a video camera (DVC 1300C:19130 RGB camera with Navitar TV Zoom 7000 zoom lens) placed 14 mm above the lens platform. The background scatter is subtracted from the scatter of the lens by subtracting an image of a blank cell using EPIX XCAP V 1.0 software. The -subtracted scattered light image is quantitatively analyzed, by integrating over the central 10 mm of the lens, and then comparing to a CSI Thin Lens®, (CSI Flexible Wear (crotofilcon A) lot ML 62900207 Power -1.0) which is arbitrarily set at a haze value of 100. Four lenses are analyzed and the results are averaged to generate a haze value as a percentage of the standard CSI lens.

Silver releasing compounds may be added in a number of ways depending upon the silver releasing compound selected. Some silver releasing compounds may be added (prior to curing) to the soft contact lens formulation. Suitable example include those described in US 5,710,302, WO 9421698, EP 406161, JP 2000016905, U.S. 5,998,498, US Pat. App. No. 09/532,943, U.S. 6,087,415, U.S. 5,760,100, U.S. 5,776, 999, U.S. 5,789,461, U.S. 5,849,811, and U.S. 5,965,631. In addition, silver releasing compounds of the invention may be added to the formulations of commercial soft contact lenses. Examples of soft contact lenses formulations include but are not limited to the formulations of etafilcon A, genfilcon A, lenefilcon A, polymacon, acquafilcon A, balafilcon A, and lotrafilcon A, galyfilcon A, senofilcon A, and the like. The preferable contact lens formulations are etafilcon A, balafilcon A, acquafilcon A, lotrafilcon A, senofilcon A, galyfilcon A and silicone hydrogels, as prepared in U.S. 5,998,498, US Pat. App. No. 09/532,943, a continuation-in-part of US Pat App. No. 09/532,943, filed on August 30, 2000, WO03/022321, U.S. 6,087,415, U.S. 5,760,100, U.S. 5,776, 999, U.S. 5,789,461, U.S. 5,849,811, and U.S. 5,965,631.

Hard contact lenses are made from polymers that include but are not limited to polymers of poly(methyl) methacrylate, silicone acrylates, silicone acrylates, fluoroacrylates, fluoroethers, polyacetylenes, and polyimides, where the preparation of representative examples may be found in JP 200010055, JP 6123860 and U.S. 4,330,383. Intraocular lenses of the invention can be formed using known materials. For example, the lenses may be made from a rigid material including, without limitation, polymethyl methacrylate, polystyrene, polycarbonate, or the like, and combinations thereof. Additionally, flexible materials may be used including, without limitation, hydrogels, silicone materials, acrylic materials, fluorocarbon materials and the like, or combinations thereof. Typical intraocular lenses are described in WO 0026698, WO 0022460, WO 9929750, WO 9927978, WO 0022459, and JP 2000107277. U.S. 4,301,012; 4,872,876; 4,863,464; 4,725,277; 4,731,079. Silver releasing compounds may be added to hard contact lens formulations and intraocular lens formulations in the same manner (prior to curing) as soft contact lenses.

The polymer used to make the ophthalmic device also influences the k value for the silver releasing compound. For example, the k value may increase with increasing equilibrium water content of water containing polymers, such as hydrogels. The solubility and/or affinity of the silver releasing compound for the lens polymer and its components may also influence k values.

Preferably the silver releasing compounds are added to silicone hydrogel formulations or contact lenses made therefrom. A silicone-containing component is one that contains at least one [—Si—O—Si] group, in a monomer, macromer or prepolymer. Preferably, the Si and attached O are present in the silicone-containing component in an amount greater than 20 weight percent, and more preferably greater than 30 weight percent of the total molecular weight of the silicone-containing component. Useful silicone-containing components preferably comprise polymerizable functional groups such as acrylate, methacrylate, acrylamide, methacrylamide, N-vinyl lactam, N-vinylamide, and styryl functional groups. Examples of silicone components which may be included in the silicone hydrogel formulations include, but are not limited to silicone macromers, prepolymers and monomers. Examples of silicone macromers include, without limitation, polydimethylsiloxane methacrylated with pendant hydrophilic groups as described in United States Patents Nos. 4,259,467; 4,260,725 and 4,261,875; polydimethylsiloxane macromers with polymerizable functional group(s) described in U.S. Patents Nos. 4,136,250; 4,153,641; 4,189,546; 4,182,822; 4,343,927; 4,254,248; 4,355,147; 4,276,402; 4,327,203; 4,341,889; 4,486,577; 4,605,712; 4,543,398; 4,661,575; 4,703,097; 4,837,289; 4,954,586; 4,954,587; 5,346,946; 5,358,995; 5,387,632 ; 5,451,617; 5,486,579; 5,962,548; 5,981,615; 5,981,675; and 6,039,913; polysiloxane macromers incorporating hydrophilic monomers such as those described in U.S. Patents Nos. 5,010,141; 5,057,578; 5,314,960; 5,371,147 and 5,336,797; macromers comprising polydimethylsiloxane blocks and polyether blocks such as those described in U.S. Patents Nos. 4,871,785 and 5,034,461, combinations thereof and the like. All of the patents cited herein are hereby incorporated in their entireties by reference.

The silicone and/or fluorine containing macromers described in U.S. Patents Nos. 5,760,100; 5,776,999; 5,789,461; 5,807,944; 5,965,631 and 5,958,440 may also be used. Suitable silicone monomers include tris(trimethylsiloxy)silylpropyl methacrylate, hydroxyl functional silicone containing monomers, such as 3-methacryloxy-2-hydroxypropyloxy)propylbis(trimethylsiloxy)methylsilane and those disclosed in WO03/22321, and mPDMS containing or the siloxane monomers described in U.S. Patents Nos. 4,120,570, 4,139,692, 4,463,149, 4,450,264, 4,525,563; 5,998,498; 3,808,178; 4,139,513; 5,070,215; 5,710,302; 5,714,557 and 5,908,906.

Additional suitable siloxane containing monomers include, amide analogs of TRIS described in U.S. 4,711,943, vinylcarbamate or carbonate analogs decribed in U.S. 5,070,215, and monomers contained in U.S. 6,020,445, mono-methacryloxypropyl terminated polydimethylsiloxanes, polydimethylsiloxanes, 3-methacryloxypropylbis(trimethylsiloxy)methylsilane, methacryloxypropylpentamethyl disiloxane and combinations thereof.

Often lenses are coated to increase their compatibility with living tissue. Therefore, the lenses of the inventions may be coated with a number of agents that are used to coat lenses. For example, the coating procedures, compositions, and methods of WO03/11551, U.S. 6,087,415, 5,779,943, 5,275,838, 4,973,493, 5,135,297, 6,193,369, 6,213,604, 6,200,626, and 5,760,100 may be used and these applications and patents are hereby incorporated by reference for those procedures, compositions, and methods. However, it is a benefit of the present invention that desirable release characteristics may be achieved without the use of coatings meant to slow the release of the silver.

Still yet further, the invention includes a method of reducing the adverse events associated with microbial colonization on a lens placed in the ocular regions of a mammal comprising, consisting of, or consisting essentially of, placing an antimicrobial lens comprising a metal releasing compound on the eye of a mammal. Preferably the lens is worn for at least 2 days of daily or continuous wear. The terms lens, antimicrobial lens, and metal releasing compound all have their aforementioned meanings and preferred ranges. The phrase "adverse events associated with microbial colonization" include but are not limited to contact ocular inflammation, contact lens related peripheral ulcers, contact lens associated red eye, infiltrative keratitis, microbial keratitis, and the like. The term mammal means any warm blooded higher vertebrate, and the preferred mammal is a human.

Still yet even further, the invention includes a method of producing an antimicrobial lens comprising, consisting essentially of, or consisting of a silver releasing compound wherein the method comprises, consists essentially of or consists of mixing the silver releasing compound with a lens formulation prior to curing. The terms antimicrobial lens and silver releasing compound have their aforementioned meanings and preferred ranges. The term "formulation" refers to any ingredient or combination of ingredients that is used to make antimicrobial lenses, such as monomers, pre-polymers, co-polymers, macromers initiators, pigments, dyes and the like. Examples of such ingredients are known in the art and some of those ingredients are disclosed in the ophthalmic lens patents and patent applications cited earlier in this application.

The lenses of the present invention may be made by numerous methods, such as the method comprising, consisting essentially of, or consisting of

(a) mixing a salt precursor, a silver releasing compound, or a combination thereof with an lens formulation;
(b) forming the lens; and
(c) when a salt precursor is used, treating the lens with a silver agent.

The terms antimicrobial lens, silver releasing compound, and lens formulation all have their aforementioned meanings and preferred ranges. The term "salt precursor" refers to any compound or composition (including aqueous solutions) that contains a cation that may be substituted with silver ions. It is preferred that the salt precursor is soluble in lens formulation at about 1μg/mL or greater. The term does not include zeolites as described in WO03/11351, solid silver as described in WO02/62402. The preferred amounts of salt precursor in the lens is about 0.00001 to about 10.0 weight percent, more preferably about 0.0001 to about 1.0 weight percent, most preferably about 0.001 to about 0.1 weight percent based upon the total weight of the monomer composition. Examples of salt precursors include but are not limited to inorganic molecules such as sodium chloride, sodium iodide, sodium bromide, lithium chloride, lithium sulfide, sodium sulfide, potassium sulfide, sodium tetrachloro argentate, and the like. Examples of organic molecules include but are not limited to tetra-alkyl ammonium lactate, tetra-alkyl ammonium sulfate, quaternary ammonium halides, such as tetra-alkyl ammonium chloride, bromide or iodide. The preferred precursor salt is sodium iodide.

The term "forming" refers to any of a number of methods used to form lenses that include but are not limited to curing with light or heat. The lens formulations of the present invention can be formed by any of the methods know to those skilled in the art, such as shaking or stirring, and used to form polymeric articles or devices by known methods.

For example, the ophthalmic devices of the invention may be prepared by mixing reactive components and any diluent(s) with a polymerization initator and curing by appropriate conditions to form a product that can be subsequently formed into the appropriate shape by lathing, cutting and the like. Alternatively, the reaction mixture may be placed in a mold and subsequently cured into the appropriate article.

Various processes are known for processing the lens formulation in the production of contact lenses, including spincasting and static casting. Spincasting methods are disclosed in U.S. 3,408,429 and 3,660,545, and static casting methods are disclosed in U.S. 4,113,224 and 4,197,266. The preferred method for producing contact lenses of this invention is by molding. For this method, the lens formulation is placed in a mold having the shape of the final desired lens, and the lens formulation is subjected to conditions whereby the components polymerize, to produce a lens. The lens may be treated with a solvent to remove the diluent and ultimately replace the diluent with water. This method is further described in U.S. Pat. Nos. 4,495,313; 4,680,336; 4,889,664; and 5,039,459, incorporated herein by reference. The preferred method of curing is with radiation, preferably UV or visible light, and most preferably with visible light.

The term "silver agent" refers to any composition (including aqueous solutions) containing silver ions. Examples of such compositions include but are not limited to aqueous or organic solutions of silver nitrate, silver triflate, or silver acetate, where the concentration of silver agent in solution is about 1 μg/mL or greater. The preferred silver agent is aqueous silver nitrate, where the concentration of silver nitrate is the solution is greater than or equal to about 0.0001 to about 30 weight percent, more preferably about greater than 0.001 to about 1 weight percent based on the total weight of the solution and, most preferably from greater than about 0.001 to about 0.03 weight percent based on the total weight of the solution. The term "treating" refers to any method of contacting the silver agent with the lens, where the preferred method is immersing the lens in a solution of the silver agent. Treating can include heating the lens in a solution of the silver agent, but it is preferred that treating is carried out at ambient temperatures. The time the lens is treated with the silver agent will depend upon the concentration of silver agent in solution and the desired initial concentration of silver in the ophthalmic device.

Yet even further, the invention includes a method of preparing an antimicrobial lens comprising, consisting essentially of, or consisting of a silver releasing compound, wherein the method comprises, consists essentially of, or consists of the steps of

(a) treating a cured lens with a salt precursor;
(b) treating the lens of step (a) with a silver agent.

The terms antimicrobial lens, salt precursor, silver agent, and treating all have their aforementioned meanings and preferred ranges.

All of the aforementioned processes may be carried out by a single mechanical device or a combination of mechanical devices. For example, if silver releasing compounds are added to cured lenses, all of the steps to add those silver releasing compounds may be carried out on a hydration machine which functions as follows. A cured lens (non-hydrated, partially hydrated or fully hydrated lens) may be placed in a single blister package. A solution of a salt precursor is added to this package and left for a time sufficient to allow the desired amount of salt precursor to be incorporated into the lens, but insufficient to produce undesirable haze. The time will vary depending upon the solubility and concentration of the salt and temperature. Suitable times (at ambient temperature) include up to about 30 minutes and preferably between about 30 seconds and 5 minutes and more preferably approximately two minutes. Subsequently, the solution of the salt precursor is removed and a solution of a metal agent is added to the package. Subsequently the metal agent solution is removed and the lens is washed with several portions of deionized water, followed by sterilization. It should be appreciated that any of the foregoing methods may include additional steps such as washing the lens, autoclaving and the like.

Still yet even further, the invention includes a method of preparing an antimicrobial lens comprising, consisting essentially of, or consisting of a silver releasing compound, wherein the method comprises, consists essentially of, or consists of the steps of

(a) treating a lens with a silver agent.
(b) treating the lens of step (a) with a salt precursor;

The terms antimicrobial lens, salt precursor, metal agent, and treating all have their aforementioned meanings and preferred ranges.

In order to illustrate the invention the following examples are included. These examples do not limit the invention. They are meant only to suggest a method of practicing the invention. Those knowledgeable in contact lenses as well as other specialties may find other methods of practicing the invention. However, those methods are deemed to be within the scope of this invention.

EXAMPLES

Silver release profiles were measured as follows: One liter of standard protein donor solution was made containing 8.80g sodium chloride, 0.46g monobasic sodium phosphate, 4.40g dibasic sodium phosphate, 1.20g bovine plasma □-globulin, 1.20g chicken egg albumin, and 1.20g chicken egg white lysozyme in deionized water. The ingredients are weighed out and placed in a 1000mL Erlenmeyer, which is then filled with deionized $H_2O$. This solution was stored in a refrigerator at 4°C throughout its use to prevent denaturing. In order to obtain release profiles, the non-hydrated lenses were placed in individual plastic vials with 2.2 mL standard protein donor solution, which was exchanged every 24 hours. The vials were kept in a tray on a plate shaker throughout the experiment, which was conducted at room temperature. Triplicate lens samples were pulled on different days throughout the thirty-day test period, dried, and analyzed for remaining silver content via INAA.

For extended antimicrobial efficacy lenses were incubated as follows: Microtiter plates for lens incubation were prepared by placing 500μL of artificial tears solution (made from 8.30g sodium chloride, 0.46g monobasic potassium phosphate, 4.40g dibasic sodium phosphate, 1.20g bovine plasma γ-globulin, 1.20g chicken egg albumin, and 1.20g chicken egg white lysozyme diluted to one liter in water) in each well of a 24-well microtiter plate. The test lenses were rinsed in three changes of 30 mis of phosphate buffered saline and transferred aseptically into individual wells of each set of microtiter plates. The microtiter plates were then placed on an orbital shaker and allowed to incubate for 24 hours at room temperature. After incubation, lenses were either transferred into the wells of new microtiter plates containing 500μL artificial tear solution or removed and tested for antibacterial efficacy as described below.

Antibacterial efficacy for initial and incubated lenses was measured as follows: A culture of *Pseudomonas aeruginosa*, ATCC# 15442 (American Type Culture Collection, Rockville, MD), was grown overnight in a tryptic soy medium. The culture was washed three times in phosphate buffered saline ( PBS, pH = 7.4 +/- 0.2) and the bacterial pellet was resuspended in 10 ml of PBS. The bacterial inoculum was prepared to result in a final concentration of

approximately 1 x 10^6 colony forming units/mL (cfu/mL). Three contact lenses were rinsed in three changes of 30 milliliters of phosphate buffered saline (PBS, pH = 7.4 +/- 0.2) to remove residual solutions. Each rinsed lens was placed with 2 mL of the bacterial inoculum into a sterile glass vial, which was then rotated in a shaker-incubator (100 rpm) for two hours at 37 +/- 2°C. Each lens was removed from the glass vial, rinsed five times in three changes of PBS to remove loosely bound cells, placed into individual wells of a 24-well microtiter plate containing 1 mL PBS, and rotated in a shaker-incubator for an additional 22 hours at 37 +/- 2°C. Each lens was again rinsed five times in three changes of PBS to remove loosely bound cells, placed into 10 mL of PBS containing 0.05% (w/v) Tween™ 80, and vortexed at 2000 rpm for 3 minutes, employing centrifugal force to disrupt adhesion of the remaining bacteria to the lens. The resulting supernatant was enumerated for viable bacteria and the results of detectable viable bacteria attached to 3 lenses were averaged and this data is presented as the log reduction of the innoculum, as compared to control (lenses made from the Table 1 formulation without added silver).

The following abbreviations were used in the Examples

Blue HEMA = the reaction product of reactive blue number 4 and HEMA, as described in Example 4 or U.S. Pat. no. 5,944,853

CGI 1850 = 1:1 (w/w) blend of 1-hydroxycyclohexyl phenyl ketone and bis (2,6-dimethyoxybenzoyl)-2,4-4-trimethylpentyl phosphine oxide

DMA = N,N-dimethylacrylamide

DPM = dipropylene glycol monomethyl ether

HEMA = 2-hydroxyethyl methacrylate

IPA = Isopropyl alcohol

mPDMS = mono-methacryloxypropyl terminated polydimethylsiloxane (MW 800-1000)

Norbloc = 2-(2'-hydroxy-5-methacrylyloxyethylphenyl)-2H-benzotriazole

ppm = parts per million micrograms of sample per gram of dry lens

PVP= polyvinylpyrrolidone (K 90)

TRIS = 3-methacryloxypropyltris (trimethylsiloxy) silane

TEGDMA = tetraethyleneglycol dimethacrylate

<u>Lens Formulation and Curing</u>

The monomer mix formulation is listed in Table 1. The preparation of the macromer is described in WO2002062402A1 (Macromer B, in Example 5) except using triethylamine to catalyze the reaction of TMI.

Table 1

|  | Weight Percent |
|---|---|
| Macromer | 17.98 |
| TRIS | 14.00 |
| DMA | 26.00 |
| MPDMS | 28.00 |
| TEGDMA | 1.00 |
| HEMA | 5.00 |
| PVP (K-90) | 5.00 |
| NORBLOC | 2.00 |
| Blue HEMA | 0.02 |
| CGI 1850 | 1.00 |

Unless otherwise specified, 3,7-Dimethyl-3-octanol was added as a diluent, with a component to diluent ratio (wt) of 80:20. Unless otherwise specified lenses were formed by placing the monomer mix in a contact lens mold and curing for 30 minutes in a $N_2$ environment at about 45°C using visible light fluorescent bulbs (Philips TL03). The lenses were hydrated using 60:40 IPA:$H_2O$ as release solution, and stepped down using 100% IPA, 80/20 IPA:$H_2O$, 60/40 IPA:$H_2O$, 40/60 IPA:$H_2O$, 20/80 IPA:$H_2O$, and 100% $H_2O$. The lenses were then rinsed in deionized water two more times and stored in deionized water.

Examples 1-4

A hydrogel blend was made from the monomer mix listed in Table 1, above, except using 30:70 (wt) dipropylene glycol:DPMA as the diluent. This blend was shipped to a commercial miller of salts along with silver chloride and silver iodide purchased from Aldrich Chemical Company. The commercial miller, ground the silver salts to a mean particle size distribution of equal to or less than 10 microns and prepared blends of the monomer mix with varying amounts of milled silver chloride (Examples 6-9) or silver iodide (Examples 1-4). Upon receipt, the resulting mixtures were rolled at 50 rpm until further use. The mixtures were loaded to an eight cavity lens mold of the type described in U.S. Patent 4,640,489. Polymerization occurred under a nitrogen purge and was photoinitiated with visible light generated with four Philips TL 03 fluorescent bulbs (4 inches above the mold), at a temperatures of 50°C over 30 minutes. After curing, the molds were opened, and the lenses were hydrated and leached by placing them successively in 40/60, 0/100, 40/60, 60/40, and 100/0 (wt) solutions of water and IPA. A minimum number of three lenses from each set were dried in a vacuum oven for three to four hours at 80°C, at a maximum pressure of five inches of Hg, and sent to an independent laboratory for residual silver content measurements by instrumental neutron activation analysis (INAA).

Silver release profiles of these lenses were measured as described above and are shown in Table 2, below. In all tables the value in parentheses after the Ag content is the standard deviation of the reported value. The values in parenthesis after the k and $[Ag]_{NR}$ values are standard errors calculated by the data fitting software.

Table 2

| Interval (days) | [Ag] (ppm) | | | |
|---|---|---|---|---|
| | Ex.1 | Ex.2 | Ex.3 | Ex 4 |
| 0 | 1213(71) | 619(15) | 322(11) | 272(9) |
| 1 | 1094(117) | 548(1) | 270(6) | 242(9) |
| 2 | 1069(95) | 512(15) | 247(3) | 216(19) |
| 5 | 949(56) | 448(8) | 207(6) | 185(12) |
| 10 | 873(127) | 349(23) | 133(4) | 106(23) |
| 15 | 628(52) | 280(5) | 85(9) | 96(7) |
| 20 | 585(53) | 225(38) | 58(4) | 69(7) |
| 25 | 608(110) | 147(22) | 40(5) | 58(8) |
| 30 | 501(175) | 120(2) | 32(1) | 73(13) |

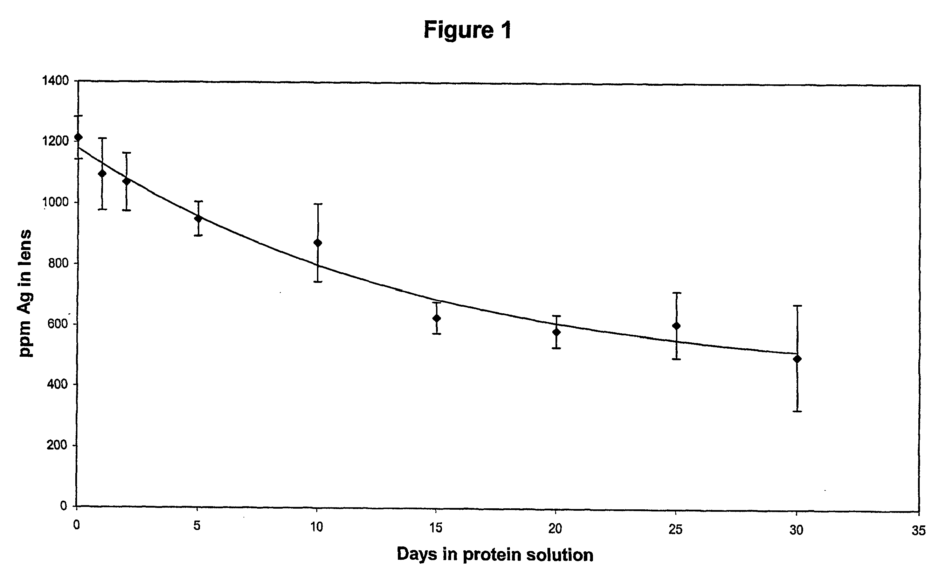

First order k values for each lens were determined are shown in Table 3. Figure 1 shows the graph of the residual silver concentration as a function of time for the lenses of Example 1.

Table 3

| | Ex 1 | Ex 2 | Ex 3 | Ex 4 |
|---|---|---|---|---|
| $[Ag]_{NR}$ ppm | 425 (103) | 0(63) | 12(12) | 58(9) |
| R squared | 0.97 | 0.99 | 0.99 | 0.99 |
| K | 0.070(0.02) | 0.052(0.01) | 0.09(0.01) | 0.13(0.02) |

Example 5

Lenses were made according to Examples 1-4, except that the initial silver content was 377 ppm. These lenses were tested for sustained efficacy using the test described above. The results are shown in Table 4, below.

Table 4

| Days in protein solution | Bacteria reduction (log) |
|---|---|
| 1 | 6.5 |
| 2 | 4.8 |

(continued)

| Days in protein solution | Bacteria reduction (log) |
|---|---|
| 10 | 4.0 |
| 15 | 4.0 |
| 18 | 3.6 |
| 25 | 3.4 |
| 30 | 3.6 |

Thus, this Example shows that lenses having AgI dispersed therein were able to substantially reduce bacterial counts when exposed to artificial tear solution throughout the entire 30 day test period.

Examples 6-9

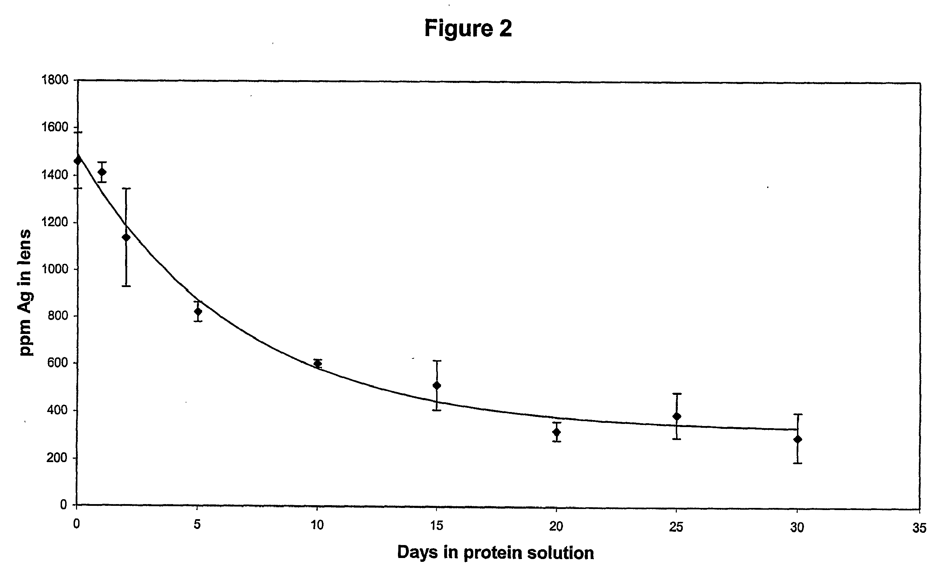

Lenses were made and analyzed for silver content according to the procedure of Examples 1-4, except using AgCl as the silver releasing compound. The results are shown in Table 5. The residual silver concentration for the lenses of Example 6 was plotted against the time interval, and is shown in Figure 2.

Table 5

| Day | [Ag] (ppm) | | | |
|---|---|---|---|---|
| | Ex 6 | Ex 7 | Ex 8 | Ex 9 |
| 0 | 1462(118) | 699(31) | 373(14) | 155(25) |
| 1 | 1413(43) | 553(136) | 382(69) | 159(14) |
| 2 | 1136(207) | 396(18) | 324(50) | 105(16) |
| 5 | 823(43) | 416(34) | 184(37) | 81(20) |
| 10 | 602(16) | 274(44) | 141(24) | 96(45) |
| 15 | 513(104) | 223(31) | 105(23) | 54(16) |
| 20 | 320(39) | 163(47) | 129(45) | 43(5) |
| 25 | 388(94) | 169(59) | 136(27) | 38(7) |
| 30 | 296(103) | 190(49) | 79(24) | 37(16) |
| $[Ag]_{NR}$ ppm | 321(47) | 182(34) | 105(17) | 35(17) |
| $R^2$ | 0.99 | 0.93 | 0.95 | 0.89 |
| k | 0.15(0.02) | 0.19(0.06) | 0.20(0.06) | 0.12(0.06) |

Example 10

Lenses were made according to Examples 6-9, except that the initial silver content was 80 ppm. These lenses were tested for sustained efficacy using the test described above. The results are shown in Table 6, below.

Table 6

| Time interval (days) | Bacteria reduction (log) |
|---|---|
| 1 | 6.5 |
| 2 | 2.4 |
| 10 | 2.1 |
| 15 | 1.3 |
| 18 | 1.2 |

(continued)

| Time interval (days) | Bacteria reduction (log) |
|---|---|
| 25 | 0.7 |
| 30 | 0.6 |

This Example shows that lenses having AgCl dispersed therein were able to substantially reduce bacterial counts when exposed to artificial tear solution throughout the entire 30 day test period. Moreover, Examples 5 and 10 show that lenses displaying a reduction in microbial colonization of at least about 2 log after about 2 and 10 days, and at least about 0.5 log after 30 days may be produced. In other embodiments lenses a reduction in microbial colonization of at least about 1 log after about 2 and 10 days may be produced.

Examples 11-14

Sodium Iodide (0.38 g, Aldrich lot# 18014BI) was dissolved in 12.25g DMA (58.9 g monomer mix). To this mixture was added the components in the appropriate amounts to form the monomer mix of Table 1. This monomer mix was degassed at 40 mm Hg at a temperature of 55°C for a total of 30 minutes. The monomer mix was used to prepare lenses using Zeonor (Zeon, grade 1060R) front curves, and Polypropylene (Fina, grade EOD 00-11) back curves. The molds had been previously spin-coated with poly(HEMA), in order to provide a mold transfer coating to the lenses, using the methods disclosed in WO03/11551. The lenses were cured under visible light (Philips TLDK-Visible-01 bulbs in a nitrogen atmosphere (<0.5 $O_2$) for 12-15 minutes @ 70$\pm$ 5°C.

The cured lenses were demolded, and immersed in a ~100 ppm silver nitrate in DI water solution for 2 hours. The hydration trays were then transferred into 60:40 IPA:DI water for 1.5 hours to release the lenses from the mold (back curve). The lenses were then swabbed into jars containing IPA. The lenses were rolled on a jar roller, and the IPA was exchanged out four times, allowing 2 hours in between exchanges. The lenses were then stepped down from neat IPA into DI water, by exchanging out: a) 10% of the IPA for DI water; b) 20% of the solution for DI water; c) 30% of the solution for DI water; d) 40% of the solution for DI water; e) 50% of the solution for DI water; f) 75% of the solution for DI water; g) 100% of the solution for DI water; h) 100% of the solution for DI water; i) 100% of the solution for DI water. The exchanges were performed at 20-minute intervals. The lenses were autoclaved in 3 mL of borate-buffered saline. The lenses were found to contain 353 $\pm$ 22 ppm silver, as measured by INAA.

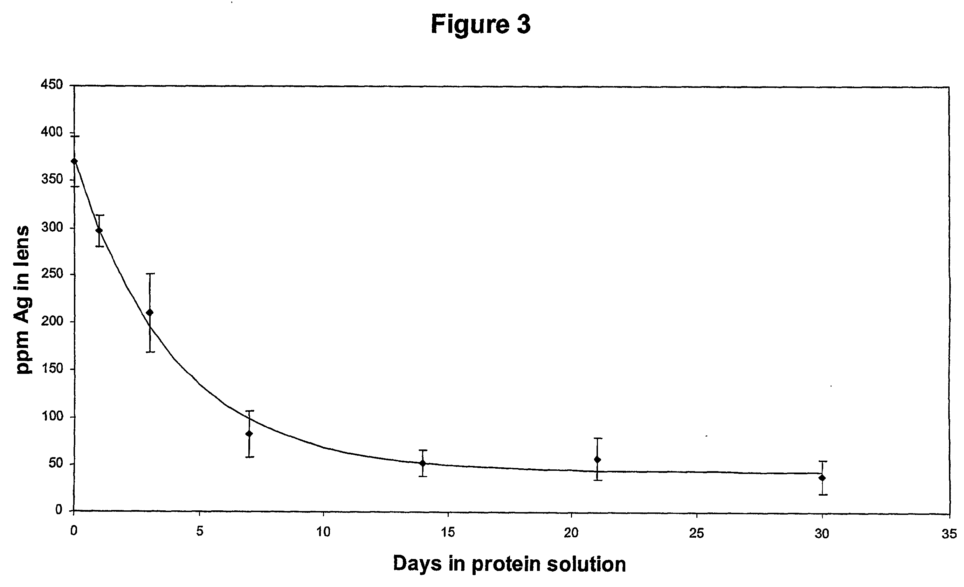

Silver release was measured using the method described above. The results are shown in Table 7. The residual silver concentration for the lenses of Example 11 was plotted against the time interval, and is shown in Figure 3.

Examples 12-14 were similarly made, except without the mold transfer coating, and with the amounts of appropriate salt (NaI, tetrabutylammonium chloride (TBACl) or tetrabutylammonium bromide (TBABr)) shown in Table 7, below added to about 15 gm of the monomer mix of Table 1.

Table 7

| | Ex 11 (AgI) | Ex 12 (AgI) | Ex 13 (AgBr) | Ex 14 (AgCl) |
|---|---|---|---|---|
| Na salt | NaI | NaI | TBABr | TBACl |
| [Na salt] (gm) | 0.38 | 0.0073 | 0.0345 | 0.0176 |
| Days | [Ag] (Std dev) in ppm | | | |
| 0 | 370 (27) | 526(21) | 1165(51) | 584(19) |
| 1 | 297 (17) | 426(7) | 156(117) | 96(49) |
| 2 | NM | NM | 115(111) | 50(18) |
| 3 | 210(41) | 297(15) | NM | NM |
| 5 | NM | NM | 28(3) | 19(0.2) |
| 7 | 82 (24) | 229(26) | NM | NM |
| 10 | NM | NM | 21(4) | 19(2) |
| 14 | 52 (14) | 86(11) | NM | NM |

(continued)

| Days | [Ag] (Std dev) in ppm | | | |
|---|---|---|---|---|
| 15 | NM | NM | 15(5) | 14(1) |
| 21 | 57 (22) | 61(11) | NM | NM |
| 30 | 38 (18) | NM | NM | NM |
| $[Ag]_{NR}$ ppm | 43 | 51 (35) | 37(21) | 37(6) |
| $R^2$ | 0.99 | 0.98 | 0.995 | 0.998 |
| k | 0.26(0.03) | 0.17(0.04) | 2.1(0.3) | 2.0(0.2) |
| NM= Not Measured | | | | |

By comparing the k values of Example 11 (0.99), with those of Examples 1-4 (k values ranging from 0.05 to 0.13) it can be seen that the method of incorporation of the silver releasing compound influences the k value. This can also be seen by comparing the k values of Example 14 (2.0) with that of Examples 6-9 (0.89-0.99). In both instances compounding the silver releasing compound into the lens formulation provided lower k values.

Examples 15

1-Vinylimidazole (4.0 g) was dissolved in 10 mL deionized water. 3.61 g $AgNO_3$ in 10 mL water was added and the resulting mixture was vigorously stirred overnight. The organic layer was dissolved in acetone and dried over $Na_2SO_4$. The acetone was removed The resulting solid product was triturated with ethyl acetate and filtered, then washed with ethyl acetate followed by hexane. The solid product was dried on a glass frit to give 2.95 g off-white product.
.This silver vinyl imidazole complex (0.2 g) was dissolved in 20.0 g of the monomer mix formulation described in Table 1. The mixture was charged into contact lens molds using front and base curves made from Topas. The lenses were cured, released and hydrated as described in the general curing conditions, above. The silver release of these lenses was measured as described above. The results are reported in Table 8. The k value was $0.20\pm0.06$, and the R2 was 0.96.

Table 8

| Day | [Ag] |
|---|---|
| 0 | 281 (26) |
| 1 | 218(22) |
| 3 | 206(43) |
| 7 | 169(32) |
| 14 | 127(7) |
| 21 | 131(3) |
| 28 | 119(16) |
| $[Ag]_{NR}$ppm | 123(11) |

Example 16

Contact lenses were made by adding 0.20 weight percent of N,N'-bis(acryloyl)cystamine (CYST) to the monomer mix of Table 1. The resulting blend was mixed for a minimum of 15 minutes to ensure even distribution of CYST in the monomer mix. The reactive monomer mix was degassed via evacuation followed by nitrogen purge, and charged into lens molds of the type described in US Patent No. 4,640,489, which had been coated with poly(2-hydroxyethyl methacrylate) as disclosed in Example 14, WO03/11551. The molds were pre-cured for $30 \pm 2$ seconds followed by cure for 12-15 minutes at $70 \pm 5°C$ under visible light (Philips TL20W/03T fluorescent bulbs). After curing, the molds were opened, and the lenses released in a 4:96 volume/volume blend of DPM and DI-water, then leached in DPM/DI-water to remove and residual monomers and diluent. The lenses were placed into vials containing 3 mL borate-buffered saline to which 50 $\mu$L of a 0.0.0315 mg/mL solution of $AgNO_3$ was added. The vials were then sealed and autoclaved for 150 minutes at 121 °C.

Silver release was measured using the method described above. The results are shown in Table 9. From the data in Table 9 the release graph was generated and $[Ag]_{NR}$ was calculated to be 57(2) ppm, the k value is 0.20(0.09) day$^{-1}$ and the $R^2$ is 0.86.

Table 9

| Days | [Ag] (ppm) |
|------|------------|
| 0 | 71(2) |
| 1 | 67(1) |
| 2 | 65(3) |
| 3 | 64(5) |
| 5 | 65(6) |
| 7 | 60(5) |
| 14 | 54(8) |
| 28 | 59(2) |

Example 17

The formulation of Table 1 (99.67 wt%) was combined with 0.33% Chelex® 100 resin (crosslinked polystyrene functionalized with the sodium salt of iminodiacetic acid groups). The mixture was charged to lens molds. Lenses were formed using the cure and hydration conditions described in the Formulation and Cure section, above. These hydrated lenses were placed into 3.0 ml/lens of a solution of AgNO$_3$ containing 13.3 ppm (wt) Ag in deionized water and autoclaved for 30 minutes at 121°C. The lenses were rinsed six times in deionized water (about 7 ml per lens), for 30 minutes per rinse. The silver release of these lenses was measured as described above. The results are reported in Table 10, below.

EXAMPLE 18

Lenses were made with 4-vinyl pyridine as described above from the formulation in Table 1, except with 0.23% (wt) 4-vinyl pyridine. The silver was measured as described above. The release results are shown in Table 10.

EXAMPLE 19

Poly(aspartic acid) (0.4029 g) in 5 Ml water was combined with 0.3714 g AgNO$_3$. This mixture was filtered and dried in vacuum at 80°C to form Ag-pASP. Lenses were made from a blend of 99.75 % (wt) the reactive monomer mixture described in the formulation section combined with 0.25% Ag-pASP. The silver release of these lenses was measured as described above. The results are reported in Table 10.

EXAMPLE 20

Lenses were made from a blend of 99.75 % (wt) the reactive monomer mixture described in the formulation section combined with 0.25% poly-L-lysine. The monomer mixture was placed in contact lens molds, cured and hydrated as described in the formulation cure section above. These hydrated lenses were placed into 3.0 ml/lens of a solution of AgNO$_3$ containing 13.3 ppm (wt) Ag in deionized water and autoclaved for 30 minutes at 121°C. The lenses were rinsed five times in deionized water (about 7 ml per lens), for 30 minutes per rinse. The silver release of these lenses was measured as described above. The results are reported in Table 10.

Table 10

| EXAMPLE | 17 | 18 | 19 | 20 |
|---------|------|------|------|------|
| Cmpd | Chelex | 4-VP | p-ASP | p-LYS |
| Days | [Ag] in ppm | | | |
| 0 | 161 (38) | 40(5) | 660(66) | 52(10) |

(continued)

| EXAMPLE | 17 | 18 | 19 | 20 |
|---|---|---|---|---|
| Cmpd | Chelex | 4-VP | p-ASP | p-LYS |
| Days | [Ag] in ppm | | | |
| 1 | 64(41) | 12(7) | 559(87) | 8(5) |
| 2 | 31(13) | 10(2) | 429(25) | 7(1) |
| 5 | 27(14) | 2(2) | 372(46) | 3(2) |
| 10 | 11(6) | 6(2) | 306(50) | 4(2) |
| | | | | |
| $[Ag]_{NR}$ ppm | 18(6) | 4.8(1.9) | 311 (30) | 4.3(1) |
| R squared | 0.99 | 0.98 | 0.98 | 0.997 |
| K | 1.1(0.2) | 1.4(0.4) | 0.42(0.12) | 2.4(0.4) |

Examples 24-27

To a solution of 50 mL methanol containing approximately 0.02 mol benzenethiol was added 100 mL of 10% (wt) $AgNO_3$. The inhomogenous mixture was left stirring for 30 minutes. The mixture was filtered and the yellow precipitate, silver benzenethiol, collected , rinsed exhaustively with MeOH and then rinsed with DI-water, and dried under vacuum at 30 °C for about 96 hours.. Silver benzenethiol (15 mg) was added to 15 gm of the monomer mix of Table 1. This mixture was sonicated for about one hour and then degassed at 40 mmHg for a total of about 30 minutes. The monomer mix formulation was loaded into an eight cavity lens mold of the type described in US Pat. No. 4,640,489 and cured for 20 to 45 minutes at a temperature of $50 \pm 5$°C. Polymerization occurred under a nitrogen purge and was photo-initiated with visible light generated with Philips TL 20W/03T fluorescent bulb.

Lenses were similarly prepared and tested using silver 2-methylbenzenethiol, silver 2-aminothiophenol, and silver thiosalicylic acid. Silver 2-methylbenzenethiol, silver 2-aminothiophenol, and silver thiosalicylic acid were all prepared in the same manner as was silver benzenethiol prepared, substituting about 0.02 mol of 2-methylbenzenethiol, 2-aminothiophenol, and thiosalicylic acid, respectively, for the about 0.02 mol of benzenethiol. All lenses were tested for silver release. The results are shown in Table 11.

Table 11

| EXAMPLE | 21 | 22 | 23 | 24 |
|---|---|---|---|---|
| Compound | benzenethiol* | 2-methylbenzenethiol* | 2-aminothiophenol* | thiosalicylic acid |
| Days | [Ag] in ppm | | | |
| 0 | 389(31) | 539(4) | 346(16) | 217(13) |
| 1 | 396(25) | 536(6) | 360(27) | 152(44) |
| 2 | 361(21) | 534(9) | 384(64) | 121(35) |
| 3 | 364(6) | 548(14) | 329(12) | 85(13) |
| 4 | 380(13) | 538(17) | 353(21) | 101(24) |
| 5 | 377(13) | 558(9) | 353(15) | 93(13) |
| | | | | |
| $[Ag]_{NR}$ ppm | 372 | 1150 | 340 | 88 |
| R squared | 0.33 | 0.45 | 0.01 | 0.97 |
| K | 0.81 | 0.0054 | 0.067 | 0.75 |

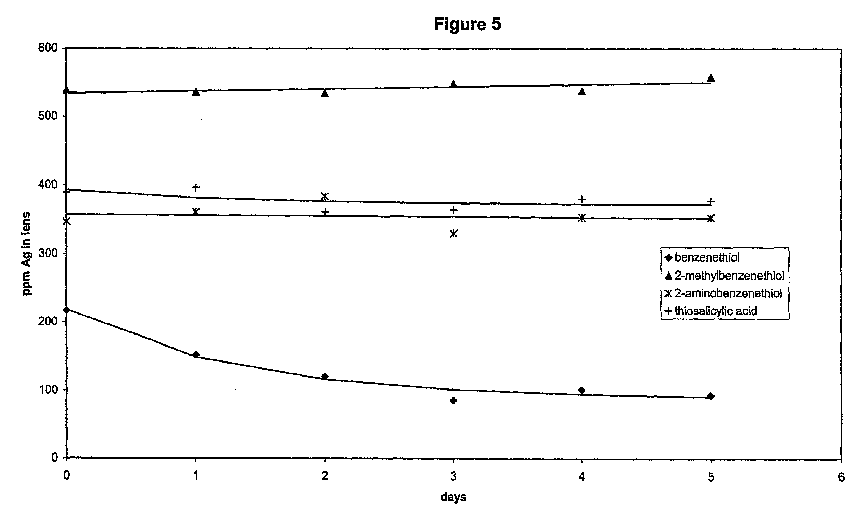

The release curves for Examples 21-24 are shown in Figure 5, which clearly shows that Examples 21-23 have very slow release profiles. This is also readily seen from the fact that the changes in silver concentration in the lenses of Examples 21-23 from day to day often do not change in an amount which exceeds the standard deviation. Because the changes in silver concentration as measured are so small and variable, the $R^2$ are very low, and in the case of Example 22, the calculated value for $[Ag]_{NR}$ does not make sense. However, the release profile clearly shows that Examples 21-23 are within the scope of the present invention. For very slow releasing materials, such as Examples 21-23, measuring the silver concentration over a period of time of at least 30 days, and preferably 45 or 60 days will provide reasonable estimates of k.

The lenses of Examples 21 through 24 were tested for initial efficacy. The results are shown in Table 12, below.

Table 12

| Ex # | Bacteria reduction (log) |
|------|--------------------------|
| 21 | 1.66 |
| 22 | 1.84 |
| 23 | 1.72 |
| 24 | 1.91 |

Example 25

Lenses were made from a blend of 99.9 % (wt) the formulation of TABLE 1 combined with 0.1% Ag(II) meso-tetraphenylporphyrin (Frontier Scientific). The silver release of these lenses was measured as described above. The results are reported in Table 13.

Table 13

| EXAMPLE | Example 28 |
|---------|------------|
| Time | |
| Days | |
| 0 | 109 |
| 1 | 97 |
| 2 | 147 |
| 3 | 96 |
| 4 | 104 |
| 5 | 123 |

Example 26

A reactive monomer mix containing 100 parts of the components shown in Table 1 and, in the amounts shown in Table 14 with 23 parts 3,7-dimethyl-3-octanol ($D_3O$). The reaction mixture was mixed vigorously (or until the solution appeared clear and evenly mixed) and then degassed, on high vacuum.

Table 14

| Component | Weight Per Cent |
|-----------|-----------------|
| Norbloc | 2.00 |
| CGI 1850 | 0.48 |
| mPDMS 1000 | 31.00 |
| DMA | 24.00 |
| HEMA | 6.00 |
| TEGDMA | 1.50 |
| SiMAA2 | 28.00 |

(continued)

| Component | Weight Per Cent |
|---|---|
| Blue HEMA | 0.02 |
| K90 | 7.00 |

The reaction mixture was then placed into thermoplastic contact lens molds and irradiated using fluorescent bulbs at 45 °C for about 15 minutes under a nitrogen atmosphere. The lenses were demolded using the procedure described above and stored in jars containing DI-water with 50 ppm of methylcellulose.

Lenses were placed into a jar containing a 1 wt. % aqueous solution of silver acetate ($CH_3CO_2Ag$, 3 mL/lens). Lenses were rolled for 60 minutes. The $CH_3CO_2Ag$ solution was decanted and a 30 wt. % aqueous solution of calcium chloride ($CaCl_2 \cdot 2H_2O$, 3 mL/lens) was added. Lenses were rolled for one hour. The $CaCl_2 \bullet 2H_2O$ solution was decanted and DI-water (3 mL/lens) was added. Lenses were rolled for one hour. The DI-water wash was repeated. Lenses were transferred individually to vials containing 3 mL of a borate buffered packing solution containing no sodium chloride and autoclaved at 121 °C for 30 minutes. Lenses were tested for silver content analysis, mechanical property testing, water content, wettability, and haze analysis. The results are shown in Table 14, below.

Control lenses were individually transferred to vials containing 3 mL of a borate buffered packing solution containing no sodium chloride and autoclaved at 121 °C for 30 minutes. Lenses were submitted for mechanical property testing, water content, wettability, and haze analysis. The results are shown in Table 15, below.

Table 15

| Property | Control Lenses | Ex. 26 |
|---|---|---|
| Water Content(%) | 39.6(0.3) | 40.5(0.5) |
| Modulus(psi) | 94(8) | 80(8) |
| Elongation(%) | 291 (32) | 216(104) |
| Tensile Strength(psi) | 147(24) | 90(50) |
| Toughness(psi) | 189(40) | 106(88) |
| Contact Angle (adv. in packing, n=5) | 44(9) | 52(17) |
| Haze (% CSI Lens) | 14(1) | 484(35) |
| Silver Content (ppm) | N/A | >1500 |

The results of Example 26 show that the treatment conditions do not provide an ophthalmic device with usable properties, particularly haze.

Example 27-29

Lenses were made as in Example 26 and treated as follows. Lenses were placed into jars containing an aqueous $CH_3CO_2Ag$ solution (0.50, 0.25, or 0.10 weight % solution, 3 mL/lens). Lenses were rolled for 60 minutes. The $CH_3CO_2Ag$ solution was decanted and a 30 wt. % aqueous $CaCl_2 \cdot 2H_2O$ solution (3 mL/lens) was added. Lenses were rolled for one hour. The $CaCl_2 \cdot 2H_2O$ solution was decanted and DI-water (3 mL/lens) was added. Lenses were rolled for one hour. The DI-water wash was repeated. Lenses were transferred individually to vials containing 3 mL of a borate buffered packing solution containing no sodium chloride and autoclaved at 121 °C for 30 minutes. Lenses were tested for silver content analysis, mechanical property testing, water content, wettability, and haze analysis. The results are shown in Table 16, below.

Table 16

| Property | Ex. 27 | Ex. 28 | Ex. 29 |
|---|---|---|---|
| % $CH_3CO_2Ag$ | 0.5wt% | 0.25wt% | 0.1 wt% |
| Water Content(%) | 40.7(0.9) | 39.1 (0.5) | 40.0(0.8) |
| Modulus(psi) | 87(11) | 87(9) | 84(5) |

(continued)

| Property | Ex. 27 | Ex. 28 | Ex. 29 |
|---|---|---|---|
| Elongation(%) | 258(53) | 301 (28) | 275(50) |
| Tensile Strength(psi) | 115(30) | 140(26) | 119(28) |
| Toughness(psi) | 140(53) | 186(42) | 150(48) |
| Contact Angle (adv. in packing, n=5) | 44(12) | 39(8) | 31(8) |
| Haze (% CSI Lens) | 232(26) | 49(13) | 15(8) |
| Silver Content (ppm) | 830(80) | 187(70) | 4.0(0.6) |

The results of Examples 27-29 show that the amount of haze may be controlled by controlling the concentration of the silver precursor. Example 10 shows that lenses made via the in situ precipitation of silver chloride display efficacy over 30 days, even at initial silver concentrations as low as 80 ppm.

<u>Preferred Features of the Application</u>

1. An ophthalmic device comprising a polymer and ionized silver in an initial concentration of at least about 10 ppm, wherein said ophthalmic device has a haze of less than about 200% and said silver releases from said ophthalmic device during use at rate with a rate constant, calculated using a first order kinetics equation, of up to about 1 days$^{-1}$

2. The ophthalmic device of paragraph 1 wherein said rate constant is between 0.001 and 0.5 days$^{-1}$, preferably between 0.01 and 0.3 days$^{-1}$, more preferably between 0.001 and 0.2 days$^{-1}$.

3. The ophthalmic device of paragraph 1 or 2 wherein said initial silver concentration is between 10 and 10,000 ppm, preferably between 25 and 5,000 ppm, more preferably between 50 and 3,000 ppm.

4. The ophthalmic device of paragraph 1 wherein said initial silver concentration and rate constant are sufficient to provide an at least about 50% reduction in microbial activity over said device's use, preferably at least a 70% reduction in bacterial activity over said use, more preferably at least a 90% reduction in bacterial activity over said use.

5. The ophthalmic device of paragraph 1 wherein said polymer is formed from a reaction mixture comprising at least one silicone-containing component.

6. The ophthalmic device of paragraph 5 wherein said reaction mixture further comprises at least one hydrophilic component.

7. The ophthalmic device of paragraph 1 wherein said ophthalmic device is coated.

8. The ophthalmic device of paragraph 1 wherein said polymer further comprises a ligand to which said silver is releasably bound.

9. The ophthalmic device of any of paragraphs 1 to 8 wherein said ophthalmic device is a contact lens.

10. The contact lens of paragraph 9 wherein said initial silver concentration and rate constant are maintained below amounts which would cause argyria.

11. The contact lens of paragraph 9 wherein after about a day said silver releases from said ophthalmic device during use at a rate with a rate constant, calculated using a first order kinetics equation of up to about 1 day.

12. The contact lens of paragraph 9 is substantially free from visible haze.

13. The contact lens of paragraph 9 having less than 150% haze, preferably less than 100% haze.

14. The contact lens of paragraph 9 wherein said use is continuous wear for at least 14 days, preferably for at least 30 days.

15. The ophthalmic device of paragraph 1 wherein said silver releasing compound has a molar solubility of silver ion in pure water of 25°C of $2.0 \times 10^{-30}$ moles/L to 2 moles/L, preferably greater than about $2.0 \times 10^{-17}$ moles/L.

16. The contact lens of paragraph 9 wherein said polymer comprises a silicone hydrogel, preferably selected from senofilcon A, galyfilcon A, lotrafilcon A and balafilcon A.

17. The contact lens of paragraph 9, wherein said lens displays a reduction in microbial colonization of at least about 2 log after two days, preferably of at least about 1 log after two days.

18. The contact lens of paragraph 9, wherein said lens displays a reduction in microbial colonization of at least about 2 log after 10 days, preferably of at least about 1 log after 10 days, more preferably of at least about 05 log after 30 days.

**Claims**

1. An ophthalmic device comprising a polymer and at least one antimicrobial metal salt comprising an initial concentration of releasable antimicrobial metal of at least about 10 ppm, wherein said ophthalmic device has a haze of less than about 200% and said antimicrobial metal releases from said ophthalmic device during use at rate with a rate constant, calculated using a first order kinetics equation, of up to about 1 days$^{-1}$.

2. The ophthalmic device of claim 1 wherein the antimicrobial metal is selected from $Mg^{+2}$, $Zn^{+2}$, $Cu^{+1}$, $Cu^{+2}$, $Au^{+2}$, $Au^{+3}$, $Au^{+1}$, $Pd^{+2}$, $Pd^{+4}$, $Pt^{+2}$, $Pt^{+4}$, and mixtures thereof.

3. The ophthalmic device of claim 1 or 2 wherein said rate constant is between 0.001 and 0.5 days$^{-1}$, preferably between 0.01 and 0.3 days$^{-1}$, more preferably between 0.001 and 0.2 days$^{-1}$.

4. The ophthalmic device of any of claims 1 to 3 wherein said initial concentration of releasable antimicrobial metal is between 10 and 10,000 ppm, preferably between 25 and 5,000 ppm, more preferably between 50 and 3,000 ppm.

5. The ophthalmic device of claim 1 wherein said initial concentration of releasable antimicrobial metal and rate constant are sufficient to provide an at least about 50% reduction in microbial activity over said device's use.

6. The ophthalmic device of claim 1 wherein said polymer is formed from a reaction mixture comprising at least one silicone-containing component.

7. The ophthalmic device of claim 6 wherein said reaction mixture further comprises at least one hydrophilic component.

8. The ophthalmic device of claim 1 wherein said ophthalmic device is coated.

9. The ophthalmic device of claim 1 wherein said polymer further comprises a ligand to which said antimicrobial metal is releasably bound.

10. The ophthalmic device of any of claims 1 to 9 wherein said ophthalmic device is a contact lens.

11. The contact lens of claim 10 wherein said initial concentration of releasable antimicrobial metal and rate constant are maintained below amounts which would cause argyria.

12. The contact lens of claim 10 wherein after about a day said antimicrobial metal releases from said ophthalmic device during use at a rate with a rate constant, calculated using a first order kinetics equation of up to about 1 day.

13. The contact lens of claim 10 is substantially free from visible haze.

14. The contact lens of claim 10 having less than 150% haze, preferably less than 100% haze.

15. The contact lens of claim 10 wherein said use is continuous wear for at least 14 days, preferably for at least 30 days.

16. The contact lens of claim 10 wherein said antimicrobial metal releases from said contact lens during use in an amount sufficient to provide at least a 70% reduction in bacterial activity over said use, preferably at least a 90% reduction in bacterial activity over said use.

17. The ophthalmic device of claim 1 wherein said antimicrobial metal has a molar solubility of antimicrobial metal ion in pure water of 25°C of $2.0 \times 10^{-30}$ moles/L to 2 moles/L, preferably greater than about $2.0 \times 10^{-17}$ moles/L.

18. The contact lens of claim 10 wherein said polymer comprises a silicone hydrogel, preferably selected from senofilcon A, galyfilcon A, lotrafilcon A and balafilcon A.

19. The contact lens of claim 10, wherein said lens displays a reduction in microbial colonization of at least about 2 log after two days, preferably of at least about 1 log after two days.

20. The contact lens of claim 10, wherein said lens displays a reduction in microbial colonization of at least about 2 log after 10 days, preferably of at least about 1 log after 10 days, more preferably of at least about 05 log after 30 days.

**Figure 1**

Figure 2

EP 1 769 809 A2

Figure 3

EP 1 769 809 A2

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 42862002 P
- US 5820918 A
- WO 0311351 A
- US 5710302 A
- WO 9421698 A
- EP 406161 A
- JP 2000016905 B
- US 5998498 A
- US 09532943 B
- US 6087415 A
- US 5760100 A
- US 5776999 A
- US 5789461 A
- US 5849811 A
- US 5965631 A
- WO 03022321 A
- JP 200010055 B
- JP 6123860 B
- US 4330383 A
- WO 0026698 A
- WO 0022460 A
- WO 9929750 A
- WO 9927978 A
- WO 0022459 A
- JP 2000107277 B
- US 4301012 A
- US 4872876 A
- US 4863464 A
- US 4725277 A
- US 4731079 A
- US 4259467 A
- US 4260725 A
- US 4261875 A
- US 4136250 A
- US 4153641 A
- US 4189546 A
- US 4182822 A
- US 4343927 A
- US 4254248 A
- US 4355147 A
- US 4276402 A
- US 4327203 A
- US 4341889 A
- US 4486577 A
- US 4605712 A
- US 4543398 A
- US 4661575 A
- US 4703097 A
- US 4837289 A

- US 4954586 A
- US 4954587 A
- US 5346946 A
- US 5358995 A
- US 5387632 A
- US 5451617 A
- US 5486579 A
- US 5962548 A
- US 5981615 A
- US 5981675 A
- US 6039913 A
- US 5010141 A
- US 5057578 A
- US 5314960 A
- US 5371147 A
- US 5336797 A
- US 4871785 A
- US 5034461 A
- US 5807944 A
- US 5958440 A
- WO 0322321 A
- US 4120570 A
- US 4139692 A
- US 4463149 A
- US 4450264 A
- US 4525563 A
- US 3808178 A
- US 4139513 A
- US 5070215 A
- US 5714557 A
- US 5908906 A
- US 4711943 A
- US 6020445 A
- WO 0311551 A
- US 5779943 A
- US 5275838 A
- US 4973493 A
- US 5135297 A
- US 6193369 B
- US 6213604 B
- US 6200626 B
- WO 0262402 A
- US 3408429 A
- US 3660545 A
- US 4113224 A
- US 4197266 A
- US 4495313 A
- US 4680336 A
- US 4889664 A

- US 5039459 A
- US 5944853 A
- WO 2002062402 A1
- US 4640489 A

**Non-patent literature cited in the description**

- Physical Chemistry. John Wiley & Sons, Inc, 300-346
- CRC Handbook of Chemistry and Physics. CRC Press, 1993
- **SKOOG, D.A. et al.** FUNDAMENTALS OF ANALYTICAL CHEMISTRY. Saunders College Publishing, 1988